# EUROPEAN PATENT APPLICATION

(11) **EP 1 365 225 A1**
(43) Date of publication of application: **26.11.2003**
(21) Application number: 03076293.4
(22) Date of filing: 29.04.2003
(51) Int. Cl.: G01M 17/02, G01N 33/00

(54) **Method and apparatus for sensing tire performance and wear**

(30) Priority: 17.05.2002 US 150674
(71) Applicant: Delphi Technologies, Inc., Troy, MI 48007 (US)
(72) Inventor: Behrendsen, Paul A., El Paso, TX 79912 (US)
(74) Representative: Denton, Michael John

(57) **Abstract**

A method and device (10) for detecting tire wear and performance in which at least one sensor device (22) is located in the interior gaseous volume (20) of a tire (12) and an associated alarm circuit (26) and transmitter (28). The device (22) is capable fo ascertaining compositional changes in the interior volume (20) of the tire (12) associated with oxidation and/or other chemical breakdown phenomena which can occur in the tire material and reporting the same as output data (33) detectable external to the tire (12).

## Description

### TECHNICAL FIELD

The present invention relates to methods and devices for sensing tire degradation. More specifically, the present invention pertains to predicting potential tire failure by monitoring physical tire characteristics including the composition of inflation gas contained in the closed environment of the tire structure.

### BACKGROUND OF THE INVENTION

There are distinct advantages to monitoring tire wear and degradation. Among these is the potential for predicting at least some tire failures during automotive operating conditions. The ability to predict at least some tire failures can enable the operator of the associated automobile to take preventative measures in a timely manner. These preventative measures may include repair and/or replacement of the tire which is at risk for failure. Such repair or replacement can ultimately enhance driver and occupant safety and prevent tire failure in an inconvenient or unsafe location.

Heretofore, most methods for predicting or preventing tire failure relied upon visual physical inspection of the tire material itself. It can be appreciated that such visual and physical inspection is, at best, a gross inspection mechanism which may reveal certain cracks, fissures, or other physical defects which are apparent on the exterior of the tire surface. Observation and identification of smaller physical defects can be more problematic. Similarly, observation and identification of defects which are located or originate on the interior of the tire material are even more difficult to carry out.

The visual defects and invisible defects referred to herein are those which typically arise as a result of routine tire wear. The material of construction of automotive tires is a synthetic polymeric material which, though extremely durable under a wide variety of operating conditions, does have a finite useful life. As the synthetic material approaches the end of its finite useful life, certain gross and microscopic structural defects begin to arise in the polymeric material itself. These gross and microscopic physical defects are the result of the chemical degradation of the synthetic polymeric material. Cracks, fissures and other material abnormalities are the physical result of this chemical breakdown.

Routine inspection of automotive tires by the automobile operator and service provider can result in repair and/or replacement of automotive tires as they approach the end of their useful life. Generally, tire manufactures promulgate recommendations regarding tire wear and number of operating miles permissible for a given tire construction. It can be appreciated that some automobile operators continue to employ tires which have exceeded the recommended useful life. Similarly, it can be appreciated that some tires are operated at more rigorous conditions which may shorten the useful life of the tire.

Thus, it would be highly desirable to provide a device and method for sensing the tire wear of each specific tire and communicating those results to the automobile operator and/or individuals who provide service for the automobile and its tires. Such information would prove useful in ascertaining and predicting the particular wear which is experienced by a given tire or tires. The information could permit more customized service and replacement of tires as required or desired and could contribute to greater occupant and operator safety by avoiding at least some unexpected tire failure events. Additionally, it could provide for the utilization and replacement of tires in a more precise manner consistent with the approaching end of useful life of the given tire thereby contributing to the more efficient and economical use of tires.

Thus, it is desirable to provide a method for sensing tire degradation. It is also desirable to provide a device for sensing such tire degradation and generating useful data therefrom.

### SUMMARY OF THE INVENTION

The present invention is directed to a process for sensing degradation of at least one tire. The tire under scrutiny has a defined and isolated interior volume. The isolated interior volume contains an interior gas which, under normal operating conditions, has an essentially constant composition.

The process of the present invention comprises the steps of obtaining a baseline analysis of the concentration of at least one of the components of the interior gas contained in the interior volume of the tire. The baseline analysis may be evaluated, recorded and stored in any suitable manner which permits analysis, access and future reference.

The process also includes steps in which updated analysis of the concentration of the gaseous component in the interior of the tire is obtained. The updated analysis is also recorded and retained in a manner which permits the analysis to be evaluated, recorded and retained as required. In the process of the present invention, the updated analysis of the concentration of the at least one component of the interior gas is compared against baseline data obtained. Comparison is performed by application of a suitable mathematical algorithm capable of producing suitable output data. The output data thus produced measured by any suitable parameters to ascertain whether or not to generate a signal detectable external to the tire interior. The process can be reiterated in an ongoing manner.

The device for sensing degradation of a tire is composed of at least one sensor means communicable with the interior gas contained in the interior volume of the tire. The sensor means is capable of detecting at least one component of the interior gas. The sensor is connected to means for ascertaining change in concentration or nature of the at least one gaseous component. Typically, this analysis means is incorporated in a suitable analytical circuit which contains appropriate algorithms and memory storage capacity for performing the iterative analysis of the at least one gaseous component and determining when a significant change has occurred in data output generated by the sensor. The device also includes a transmitter associated with the sensor. The transmitter is capable of emitting a signal when triggered by the appropriate output from the sensor and associated analytical circuit. Typically, the analytical circuit is an alarm circuit at least one algorithm as well as the hardware to analyze measured data against suitable parameters. Such an alarm circuit is typically connected directly to the sensor.

The device of the present invention also includes means for producing power sufficient to operate the sensor, transmitter and data retention and analytical means. Such power production means typically includes an onboard battery associated with the preceding devices. Also, optionally present is a suitable generator for recharging the battery.

### BRIEF DESCRIPTION OF THE DRAWING FIGURES

In order to further illustrate and describe the present invention, reference is made to the following drawing figures in which:
Fig. 1 is a process diagram of the method of the present invention analyzing for at least one gaseous compound;
Fig. 2 is a process diagram of the method of the present invention analyzing against at least one gaseous compound and at least one other physical tire characteristic; and
Fig. 3 is a cross section of a tire depicting a sensor positioned therein.
Fig. 4 is a block diagram of a device for monitoring tire material condition according to the present invention.

### DESCRIPTION OF THE PREFERRED EMBODIMENT

The present invention is predicated upon the unexpected discovery that there is a correlation between tire material degradation events and changes in the gaseous environment present in the closed, isolated volume defined as the interior of the tire, hereafter "interior gas." It has been found, quite unexpectedly, that measurement of changes in the concentration of at least one gaseous component present in the interior gas can serve as an indicator of future or impending tire failure events in at least some instances.

As used herein "tire material degradation event" is to be broadly construed as any of a number of chemical reactions or interactions which can indicate or lead to a decline in at least one performance characteristic of tire material. Such declines can ultimately lead, either directly or indirectly, to material failure or substandard performance. Non-limitative examples of such tire material degradation events include oxidation, excessive localized crosslinking and the like.

Without being bound to any theory, it is believed that in at least certain tire failure events, when the tire fails, the failure has propagated from a localized anomaly present in the tire material. When the anomaly is indicated, it is believed that the initiation can occur at a molecular level in the chemical structure of the tire material. While it may be remotely possible that such anomalies are present from production of the tire material, it is believed that such anomalies largely occur as a result of the ongoing natural aging and curing process of the tire material itself over the life of the tire material.

While the anomaly may remain localized, in certain instances, it is believed that the anomaly can propagate into a region of local degradation. Given the chemical nature of many such anomalies, once a certain level of degradation has been achieved, the degradation reaction can become self-sustaining. Eventually, the local degradation phenomenon causes sufficient reduction in physical properties of the associated material to self-propagate thereby causing an often rapid general failure event of the associated tire.

It has been found that the degradation phenomenon is characterized by an exothermic reaction which can be observed as localized heating of the tire material. The exothermic reaction is due to the chemical nature of the breakdown. Exothermicity is one component which can explain the self-propagating nature of the failure event and can also explain why, after a certain point, propagation results in an often rapid progress toward a general tire failure event.

It has also been found that the degradation process can be exhibited as oxidation of the tire material. Local degradation events are characterized by changes in certain signature gaseous materials which are consumed or produced as a result of the oxidation reaction. Typically, these materials include, but are not limited to, compounds such as carbon monoxide, carbon dioxide, butylene oxides and the like. It has been found that compounds such as these are associated with the oxidative reactions in which synthetic polymeric materials employed in tire body degrade. These products of oxidation typically off-gas from the polymeric material as the reaction progresses. At least a portion of these oxidation products off gas into the volume of the interior tire chamber.

In a normally functioning tire, it has been found that the interior gas composition exists at a rough equilibrium. Depending upon the material composition of the tire, the initial interior gas composition (G_{I}) will fall within predictable baseline values after an initial interval subsequent to inflation. The occurrence of a localized tire degradation event leads to a change in gas composition (Gₛ). It has been found, quite unexpectedly, that this change in gas composition (Gₛ) can be ascertained in a manner which provides strong correlation between change in gaseous composition and tire failure events. Such tire failure events can include various forms of rupture, including slow leak and more rapid deflation phenomenon as well as more generalized tire structural failure.

The present invention is predicated upon the unexpected discovery that sensing changes in the concentration of the interior gas composition contained within the closed environment of the tire interior can be a reliable indicator of tire material quality and performance. The present invention is directed to a method and apparatus for ascertaining the quality and performance of tire material based on measurement of interior gas composition as well as measurement of optional variables such as tire pressure, temperature, etc.

The method and apparatus of the present invention are particularly directed to sensing and predicting tire performance in tires used in automotive applications. Tires so monitored may be employed on passenger vehicles as well as larger trucks, both over land and off-road. It is also contemplated that the device and method of the present invention may also be employed with tires employed on other types of vehicles, for example, airplanes.

It is contemplated that the tires so monitored may have any standard construction provided that the tire has the interior volume suitable for maintaining a gaseous composition in isolation from the surrounding atmosphere. The material of construction of such tires is generally referred to as a rubber. As used herein, "rubber" is defined as a solid substance which, upon vulcanization, becomes elastic. The term broadly includes both natural rubber (caoutchouc) and synthetic rubber. The term "rubber elastomer," as used herein, is defined as a material which contains at least one rubber synthesized from a raw material derived from at least one of petroleum, coal, oil, natural gas, and acetylene. The rubber elastomer may be made up of various copolymers. Examples include, but are not limited to, styrene-butadiene copolymers such as Buna A and styrene-butadiene rubber (SBR); cis-polybutadiene and cis-polyisoprene (butyl rubbers); copolymers of acrylonitrile and butadiene (nitrile elastomers or NBR rubbers); polychloprene (neoprene); ethylene-propylene rubbers (EPDM); and urethane elastomers.

As indicated previously, typically, tires of such construction are found on passenger automobiles. However, the process of the present invention is not to be construed as being limited simply to passenger automotive tires. The process and device of the present invention is contemplated as being employable on any vehicular tires of the same or similar construction as noted herein. Thus, in addition to passenger vehicles, it is contemplated that the process and device of the present invention can be successfully employed with larger trucks as well as off-road vehicles. Additionally, where tire construction is the same or similar to that described herein, it is contemplated that the sensors can be successfully employed for airplane tires and the like.

In the process of the present invention, the tire 12 will have a defined and isolated interior defined by inner and outer tire walls 14, 16 and wheel rim 18. Tire 12 is mounted and affixed to wheel rim 18 by any suitable sealing and fastening means as would be conventional in the field. The tire 12 will also include at least one means for introducing gas into the defined and isolated interior volume such as an inflation nipple (not shown). Typically, under both operational and non-operational states, when the tire is employed on an automotive vehicle, the communication means is sealed preventing ingress and egress of gaseous material from the defined and isolated interior volume 20.

In standard use operations, the tire is pressurized to a predetermined pressure rating. Thus, the air pressure of the gaseous material located in the defined and isolated interior volume is greater than ambient. In most automotive situations, tire pressure pressurization is accomplished by the introduction of pressurized atmospheric air. Typically, atmospheric air contains approximately 70% nitrogen; approximately 20% oxygen and about 5% and 10% carbon dioxide with traces of other gaseous material being present to amount to 100%.

Upon initial inflation, atmospheric air introduced into the tire interior reaches compositional equilibrium with any gaseous components introduced as a result of natural off-gassing by the rubber elastomer of the newly produced tire. These additional gaseous components can include production by-products and volatilizable solvents in present in minor amounts in the elastomeric rubber as processed. The interior gas present in the defined and isolated interior volume typically reaches an equilibrium composition which may contain minor amounts of various organic processing by-products. It is to be understood that such processing by-products are generally those which do not adversely affect the process and function of the device of the present invention.

Routine tire repressurization or introduction of additional atmospheric air to correct tire inflation is contemplated and anticipated. Introduction of additional atmospheric air typically will not effect gas equilibrium in a manner which will adversely effect performance or implementation of the method or device of the present invention.

While the present invention is described in view of tire pressurization using atmospheric air, it is to be understood that the method and device of the present invention can be employed using any suitable inflation gas.

In the process of the present invention, a baseline quantification or analysis of at least one component of the interior gas contained in the isolated tire interior 20 is obtained. The analysis contemplated herein can be any suitable qualitative or quantitative analytical procedure which ascertains presence, absence and/or concentration of at least one component of the interior gas. In the process of the present invention, it is preferably contemplated that the ascertainment analysis will be accomplished by at least one suitable analytical means such as a suitably calibrated sensor mechanism 22. The analytical means is capable of communicating and/or contacting interior gas material present in the isolated interior volume.

The analytical means is preferably one which can be calibrated to detect at least one component such as oxygen, carbon monoxide, carbon dioxide and butylene oxide. It is also within the purview of this invention to detect various trace organic compounds. The analytical means may be equipped to simply measure presence or absence of a given substance or may be used to provide more detailed information regarding relative concentration of a target material or materials. In the preferred embodiment, the analytical means is a sensor mechanism which is configured to detect and/or quantify the presence of at least one target gas. As used herein, the term "sensor mechanism" is contemplated as encompassing a device or probe capable of sensing one specific material or configured to provide information regarding a plurality of target materials by any suitable analytical modality. Such modalities include, but are not limited to, infrared spectral analysis, ultraviolet spectral analysis as well as various other methods for detecting and quantifying organic and inorganic compounds. Suitable sensor mechanisms for sensing the presence of a single material or multiple materials are commercially available from various sources.

In the process and device of the present invention, sensor mechanisms are typically positioned so as to be in contact with the interior gas. "Contact" as used herein can be continuous contact and can also include metered or interval contact where the gaseous material is sampled and a discrete sample is analyzed and data extrapolated therefrom.

In the process of the present invention, baseline compositional data regarding the content and makeup of the interior gas is obtained. As used herein, "baseline gaseous composition data" can include standard values which are predetermined and/or obtained from initial measurement of the interior gas. Thus, the method of the present invention contemplates the use of standard preprogrammed baseline gaseous composition values. It also contemplates a process whereby initial startup values for the gaseous composition are obtained and employed in determining baseline data regarding the interior gas as well as a combination of the two.

Preprogrammed gaseous compositional values can be maintained in any suitable data retention device such as an electronic microchip or the like. The term "preprogrammed gaseous compositional values" as used herein is contemplated as referring to accepted concentration ranges for a given gaseous component or components. The preprogrammed gaseous compositional values can alternatively relate to accepted upper or lower threshold limit for the gaseous component under consideration. Baseline gaseous composition data can also include information derived from sensor analysis at start up. This information can be retained for subsequent comparison and/or analysis. The information can also be compared against preprogrammed gaseous compositional values to determine whether acceptable thresholds have been met.

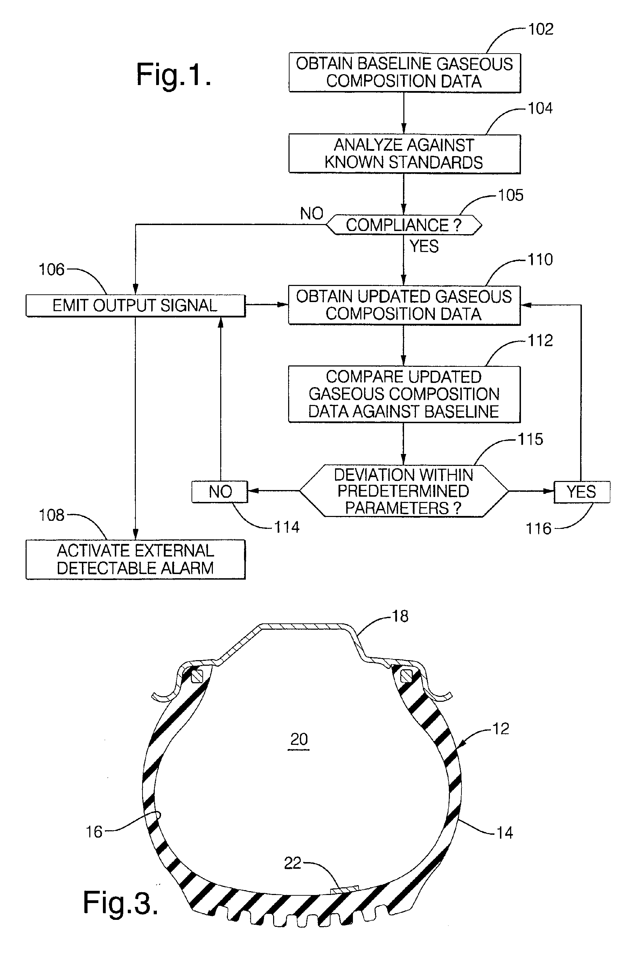

In the basic process of the present invention, as depicted in Fig. 1, baseline gaseous composition data includes use of preprogrammed standards as well as sample analysis at startup. As depicted in step 102, data generated at start up can be analyzed against known or preprogrammed standards which are taken to define compliance. Compliance with known or preprogrammed standards may be accomplished by any suitable comparison algorithm at step 104. Comparison and analysis against known standards as depicted in step 105 can yield a simple yes/no conclusion. If compliance standards are violated, an alarm mechanism is activated which will result in the emission of an output signal at step 106 detectable external to the tire.

In the process of the present invention, where baseline compositional analysis is employed, it is preferred that at least one chemical compound associated with the degradation process be monitored and tracked. This chemical compound may be one which is present in elevated quantities upon occurrence of oxidation or degradation phenomena. It is also within the purview of this invention that the chemical compound monitored be one which is consumed during degradation and/or oxidation processes. Thus, baseline analysis against known or predetermined threshold values can be quantified as concentrations of a chemical which either falls below or exceeds accepted operating values.

In the event of deviation from preprogrammed baseline values is detected, step 108 of the process of this invention contemplates the generation and transmission of a suitable signal which is detectable external to the tire being monitored. This signal may be in any suitable form such as electric impulse, carrier wave or the like and may be translated into any suitable audible or visually detectable warning. Typically, such warning will be one which will be readily detectable and intelligible by the vehicular operator. However, it is also within the purview of this invention that the signal may be transmitted to suitable external diagnostic devices as would be present in a vehicular service center or the like.

It is to be understood that the present invention contemplates the analysis of at least one chemical compound. Greater numbers of chemical compounds can be monitored depending upon the configuration of the sensor or sensors employed.

Where calculations have indicated that the values obtained are considered to be in compliance, the monitoring process is continued. Updated analysis of the at least one chemical compound present in the gaseous composition is obtained at predetermined intervals at step 110 after start up. The predetermined intervals can be any period suitable for providing appropriate monitoring of tire condition and wear. It is believed that intervals between 25 seconds and 10 minutes are sufficient to provide indication of tire performance.

The process of the present invention also contemplates the additional analysis at step 112 of the updated data pertaining to the at least one chemical compound to determine deviation from baseline. Where deviation between the updated data and the predetermined baseline values falls outside predetermined thresholds shown in step 114, the suitable circuit is triggered to emit an output signal at step 106. Where deviation by updated gaseous composition data from the baseline data is within predetermined parameters, the iterative interval process is continued, as shown in step 116.

It is contemplated that the process of the present invention as depicted in Fig. 1 will occur in an essentially continuous manner during operation of the associated vehicle. Thus, preferably, sensor operation and output development is terminated when the vehicle is not in operation. Thus, it is contemplated that the control mechanisms for executing suitable analytical routines associated with the process of the present invention will include suitable shut down procedures and the like. Where the process of the present invention contemplates diagnostic output to an external service center location, such as an auto repair facility, it is contemplated that the process and devices for implementing the process will include appropriate programming and associated hardware to facilitate the transfer of relevant information.

In the process of the present invention, it is contemplated that monitoring of the at least one chemical compound will continue even after an output signal at step 106 has been generated. Thus, updated data will be obtained even in situations where a deviation has been indicated. Continued monitoring and iteration after indication of an initial deviation can be employed to assess the severity of the situation and permit self-correcting measures. Erroneous or non-homogenous false positives can be corrected upon analysis of subsequent sample iterations. Additionally, output data may be employed to initiate appropriate self-healing or self-correcting processes within the tire which may ultimately extend tire life.

While the process of the present invention may be performed with analysis and monitoring of one chemical compound, analysis of multiple chemical compounds can be desirable to provide greater accuracy in the assessment of tire wear. Thus, it is contemplated that suitable sensor operations preferably will be those which can monitor and provide information regarding several distinct chemical compounds present in the gaseous material in the isolated interior of the tire.

The compounds suitable for analysis in the process of the present invention are those which are gaseous or volatilizable and are capable of detection by suitable analytic sensor mechanisms. Typically, such compounds are amenable to detection and/or quantification by methods known and associated with gaseous analysis and detection. These include, but are not limited to, infrared and ultraviolet spectral analysis. Chemical compounds of interest include, but are not limited to, inorganic gasses such as carbon dioxide, carbon monoxide, oxygen and the like as well as volatile organic compounds such as oxygen derivatives of alkylene and aryl alkylenes. Examples of volatile organic compounds include, but are not limited to, butylene oxide, propylene oxide and the like. It is also within the purview of this invention that the at least one chemical compound is a material specifically introduced into the inflation gas as a detectable and discrete indicator of tire wear. Suitable indicators include those which are preferentially consumed or altered during degradative processes as well as those which may interact with materials generated to provide ready detection by sensor mechanisms. Thus, decrease in oxygen content taken together with an associated increase in concentrations of a compound such as butylene oxide can be indicative of an oxidative chemical breakdown in an isolated region of the tire material. Thus, while the method depicted in Fig. 1 has been described in terms of a single chemical compound, it is to be understood that multiple materials can be analyzed in tandem to provide more detailed or complex data regarding the general condition of tire material and wear of the tire.

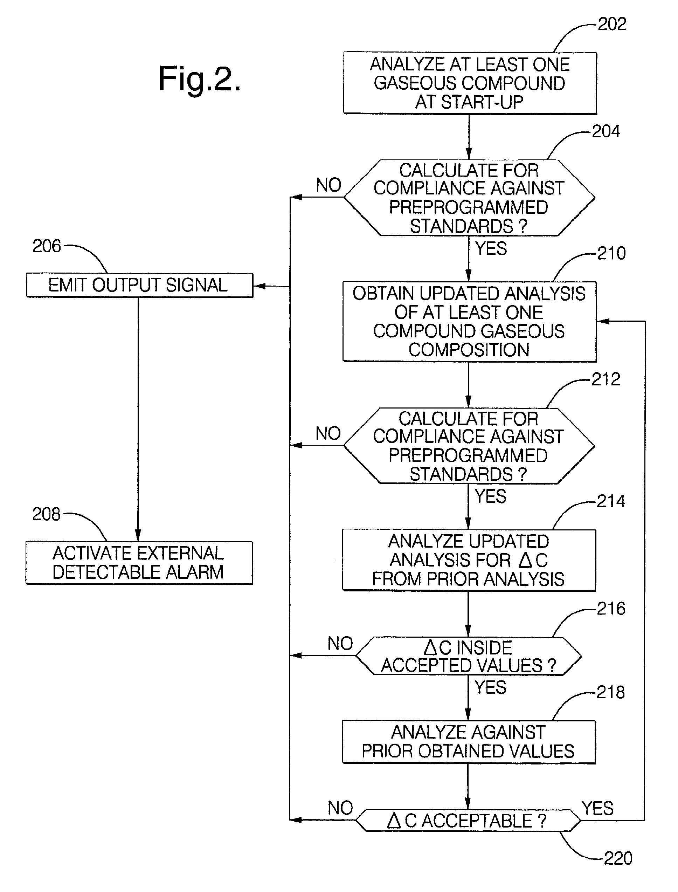

The process of the present invention also contemplates an analytical procedure in which at least one chemical compound is monitored for both deviation or change in value over time and for compliance and/or deviation from preprogrammed baseline standards as outlined in Fig. 2. In this embodiment of the analytical method of the present invention, initial analysis of at least one chemical compound is taken at cycle startup step 202. This data is calculated against compliance standards which are preprogrammed into the analytical mechanism employed 204. If compliance is violated, an output signal is emitted at step 206. The output signal is translated into activation of an externally detectable alarm at step 208 such as in the manner described previously. If the at least one chemical compound is analyzed and calculated to be within preprogrammed standards, monitoring proceeds with performance updated analysis of chemical compound at a suitable interval subsequent to the startup value as shown at step 210. As indicated previously, this interval can be any one suitable for providing pertinent data regarding the condition of the associated tire material. The minimum interval may be that which is determined by the recalibration or reset limits present on the associated sensor device, processing unit or other mechanisms in the alarm circuit. Typically, the updated analysis may occur within microseconds of the startup analysis or may occur at intervals between 1 second and 10 minutes.

The updated analytical data of at least one chemical compound is calculated for compliance against preprogrammed standards at step 212. As previously discussed, deviation from compliance results in the emission of an output signal at step 206. Where analytical data is determined to be within appropriate standards, the data is further quantified at step 214 to determine the amount of change in the values obtained in the updated analysis and at least one preceding analysis to determine the degree of change (c) and/or rate of change (cᵣ) which is occurring.

At step 216, the c value obtained is mathematically compared against acceptable c values at step 216 which are maintained in suitable data storage means. If the obtained c deviates from the accepted value parameters, an output signal is emitted at step 206. Maintenance of a c within accepted preprogrammed can trigger analysis of the most recent c against any and all prior obtained c values to determine general trend or rate of change(cᵣ) at step 218. If the value for the rate of change (cᵣ) deviates from predetermined c values, an output signal is emitted at step 206. If the cᵣ values are within acceptable parameters 220, additional samples are obtained after at appropriate intervals at step 210.

While the foregoing process has been described in terms of analysis for a single chemical compound, it is to be understood that the process of the present invention can readily include analysis for compliance and/or deviation for multiple chemical materials. Thus, for example, sensors can be calibrated to detect the presence, absence and/or concentration of a material such as butylene oxide, a known by-product of tire degradation. Concentrations of such material above a preprogrammed standard can trigger the output signal event of the present invention.

The process and device of the present invention also contemplates the maintenance and storage of ongoing data regarding presence, absence and/or concentration of at least one chemical compound present in the interior gas over time. Thus, where desired or required, compositional changes over a given interval can be accessed and analyzed by suitable devices not typically present on an automotive vehicle.

Similarly, it is considered within the purview of this invention to integrate other non-chemical analytical data into an algorithm which leads to the generation of an output signal. For instance, the sensor array can include appropriate devices for measuring tire temperature, pressure within the internal volume of the tire, and the like. These physical, non-chemical characteristics can, then be integrated as data into the calculations and summation which has been previously described. Thus, rapid changes in internal volume pressure can be indicators of an impending tire failure event. When such data is analyzed against gaseous composition data, the certainty of the level of certainty in the prediction can be increased.

Additionally, the data which is obtained and analyzed regarding the chemical and physical conditions in the interior tire volume can be calculated against external physical or chemical data. Thus, the present invention contemplates the integration with sensors and data collection devices which are located on or in the vehicle body exterior to the tire. These can include sensing devices which monitor external temperature as well as devices which monitor tire rotational speed, vehicular speed and the like.

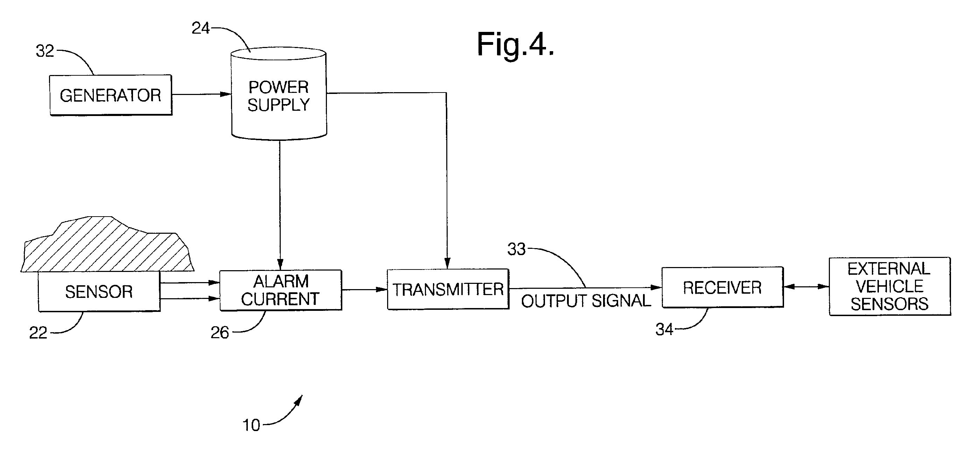

With reference now to Fig. 4, the device 10 for monitoring tire material condition typically includes at least one sensor device 22, means 24 for supplying power to the gas sensor device, a suitable alarm circuit 26 as well as a device 28 for transmitting data to a source 30 external to the associated tire.

Additionally, the device 10 can include an appropriate tire mounted generator 32 which can provide additional or continuing power to the sensor device 22, alarm circuit 26 and transmitter 28.

While the device may include a generator 30, it is also contemplated that the power source would be any suitable power source such as an appropriate battery which could be sized to last the life of the sensor 22 and associated mechanisms.

The sensor device 22 may be of any suitable type which will detect at least one chemical compound. Thus, the sensor device 22 can be one which is capable of electrochemical analysis, infrared chemical analysis, ultraviolet chemical analysis, visible spectrum analysis or a laser diode type of device capable and calibrated to sense at least one chemical compound. Preferably, the sensor is calibrated and capable of sensing more than one chemical compound with at least one of the materials be sensed includes oxygen, carbon dioxide, carbon monoxide and an alkylene oxide such as butylene oxide.

The sensor 22 can be either a quantitative sensor of a qualitative sensor. Thus, the sensor device 22 employed may be capable of analyzing and determining approximate chemical concentration within the tire interior. However, the present invention contemplates that the sensor 22 may be one which is capable of simply detecting a change in gaseous composition in the interior of the tire over time.

The alarm circuit 26 may be of any suitable configuration which is capable of maintaining and applying appropriate algorithms in conjunction with the needed hardware to perform the function of determining when a significant change has occurred in the sensor output. Typically, the alarm circuit 26 is connected directly to the sensor 22 to perform this function. The alarm circuit 26 is connected to a suitable transmitter 28 which is configured to emit a signal 33, such as a radio frequency wave or other carrier wave, when the sensor 22 has activated the circuit 26. In the preferred embodiment, sensor 22, alarm circuit 26 and transmitter 28 are all powered by the power source such as the battery 30 and optional generator 32. Typically, sensor 22, alarm circuit 26, transmitter 28 together with battery 30 and optional generator 32 are carried in and on the respective tire being monitored.

It is contemplated that the tire can be manufactured with the device in place. Alternately, the monitoring device of the present invention can be installed during any suitable point in the assembly process such as the installation of the tire 12 on the wheel rim 18. The transmitter device 28 is preferably configured to interact with a suitable receiver 34 located external to the tire 12. The suitable receiver 34 is preferably positioned in a suitable location in the body fo the associated vehicle. However, it is understood that a suitable receiver 34 can be integrated into a suitable free standing diagnostic device suitable for use by automotive maintenance personnel and the like.

The signal 33 transmitted from transmitter device 28 to receiver 34 will depend upon the nature and location of the two devices. Thus, it is contemplated that the transmitter 28 would emit a signal 33 such as a radio frequency wave when the sensor 22 has activated the alarm circuit 26. The signal 33, such as a radio frequency wave, can be detected and processed by the receiver 34. Typically, the receiver 34 will be powered by the vehicular electrical system or a suitable source of external power and will be capable of passing along the alarm signal to the driver or diagnostician via any suitable visible or audible means. It is also contemplated that the receiver 34 may include components which permit it to function interactively with other vehicular input data. Thus, the receiver 34 may include suitable capabilities which permit further analysis of the data obtained from the tire circuit device in combination with additional available vehicular data which can include information such as ambient air temperature, tire temperature, tire pressure, wheel speed, vehicular speed and the like. Alternately, it is envisioned that the external vehicular sensors can communicate directly with the alarm circuit and transmitter located on board the tire or tires to provide data for use in the appropriate algorithm.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims. Accordingly, it is to be understood that the present invention has been described by way of illustration and not limitation.

## Claims

1. A process for sensing performance of at least one tire (12), the tire having a defined and isolated interior (20) which contains a discrete volume of a gas, the gas having an essentially constant composition, the process comprising the steps of:
obtaining a baseline analysis of at least one chemical compound present in the interior volume of the gas (102);
obtaining at least one updated analysis of the at least one chemical compound present in the interior volume of gas at at least one interval subsequent to the baseline analysis (110);
comparing the baseline analysis and the at least one updated analysis using a suitable mathematical algorithm which will function to provide output data (112);
applying said output data against preprogrammed standards to generate a signal (115).

2. The process of claim 1 wherein the generated signal is transmitted to an output device.

3. The process of claim 1 wherein said generated signal triggers a reiteration of the analytical process.

4. The process of claim 1 wherein the at least one chemical compound includes at least one of alkylene oxide, oxygen, carbon dioxide, and carbon monoxide.

5. The process of claim 1 wherein the at least one chemical compound material is alkylene oxide and the output signal is triggered upon detection of an elevation in butylene oxide concentration in the essentially interior volume of gas.

6. The process of claim 5 wherein the at least one gaseous material further includes oxygen and wherein the output signal is determined by a reduction in oxygen concentration in the essentially constant composition of the interior volume of the tire.

7. The process of claim 1 further comprising the step of integrating data pertaining to at least one physical condition external to the tire volume into the algorithm.

8. The process of claim 8 wherein the at least one physical condition external to the interior tire volume includes at least one of tire pressure, tire temperature, wheel speed, ambient temperature and vehicular speed.

9. A device (10) for sensing degradation of a tire (12) having an isolated interior (20) which contains a discrete volume of an essentially constant gaseous composition, the device (10) comprising:
at least one sensor device (22) capable of detecting at least chemical compound, the chemical compound including at least one of oxygen, carbon dioxide, carbon monoxide, alkylene oxide, the sensor (22) positioned relative to the isolated interior of the tire in gaseous communication therewith;
an alarm circuit (26) in communication with the sensor device (22), the alarm circuit (26) containing an appropriate algorithm and suitable means for receiving data generated by the sensor device and interpreting data so received;
means (24) for supplying power to the sensor device and associated alarm circuit (26); and
a transmitter (28) in communication with the alarm circuit (26), the transmitter (28) capable of emitting an output signal (33), the output signal (33) being detectable external to the tire (12).

10. The device of claim 9 further comprising means for generating power sufficient to operate the sensor device and alarm circuit.

11. The device of claim 9 wherein the alarm circuit is capable of ascertaining significant change in sensor output over time.

12. The device of claim 9 further comprising a receiver mechanism located external to and remote from the transmitter and alarm circuit, the receiver capable of detecting the signal generated by the transmitter and translating said signal into a detectable alarm event.

13. The device of claim 12 wherein the receiver mechanism includes functionality for processing additional input, the additional input including at least one of ambient air temperature, tire temperature, tire pressure, wheel speed, vehicular speed and combinations thereof.
